# FASCICULE DE BREVET EUROPEEN

(11) **EP 0 880 340 B1**
(45) Date de publication et mention de la délivrance du brevet: **08.10.2003**
(21) Numéro de dépôt: 97903433.7
(22) Date de dépôt: 10.02.1997
(51) Int. Cl.: A61B 17/16, A61F 2/46

(54) **INSTRUMENT DE POSE DE PROTHESE DE HANCHE**
INSTRUMENT ZUM EINSETZEN EINER HÜFTPROTHESE
HIP PROSTHESIS POSITIONING INSTRUMENT

(30) Priorité: 13.02.1996 FR 9602002
(43) Date de publication de la demande: 02.12.1998
(73) Titulaire: ADVANCED TECHNICAL FABRICATION, 74970 Marignier (FR)
(72) Inventeur: BRESLER, Franck, F-54000 Nancy (FR); CATIER, Philippe, F-35740 Pace (FR); CAUDAL, Philippe, F-83420 La Croix-Valmer (FR); FRANCOIS, Jean-Marie, F-67500 Marienthal (FR); GODEFROY, Jean, F-74130 Ayze (FR); HOROSZOWSKI, Henri, décédé (IL); MOLE, Daniel, F-54000 Nancy (FR); RIVAT, Paul, F-07130 Saint-Peray (FR)
(74) Mandataire: Poncet, Jean-François
(86) Numéro de dépôt international: FR9700257
(87) Numéro de publication internationale: WO97029698

(56) Documents cités:
- EP-A- 0 051 359
- EP-A- 0 147 339
- EP-A- 0 327 509
- EP-A- 0 470 912
- EP-A- 0 578 322
- EP-A- 0 704 191
- DE-A- 3 903 832
- FR-A- 2 281 095
- US-A- 4 712 951

## Description

La présente invention concerne les instrumentations utilisées pour poser les prothèses de hanche, et en particulier pour poser les implants cotyloïdiens formant la partie femelle de l'articulation de hanche.

Dans les techniques opératoires habituellement utilisées pour poser un implant cotyloïdien, la première opération consiste à fraiser une cavité hémisphérique dans le cotyle osseux, au moyen d'une fraise hémisphérique. Une telle fraise hémisphérique est décrite par exemple dans le document EP-A-0 704 191 publié après la date de priorité, et comporte une calotte sensiblement hémisphérique creuse avec des lumières à bord coupant et une base ouverte dans laquelle sont prévues des entretoises radiales sur lesquelles peut se fixer un porte-fraise. Le porte-fraise est un manche avec un tube externe de manutention dans lequel tourillonne un tube de manoeuvre lui-même monté à coulissement longitudinal sur un arbre d'entraînement. L'extrémité de l'arbre d'entraînement porte une douille à baïonnette, comportant des encoches d'extrémité en L réparties sur le bord extrême de la douille et conformées pour recevoir et retenir les entretoises radiales de la fraise. Le tube de manoeuvre est solidaire d'une platine coulissante portant des ergots de verrouillage. Un ressort repousse le tube de manoeuvre et la platine coulissante en appui contre la douille à baïonnette, position dans laquelle les ergots de verrouillage ferment les encoches en L pour emprisonner les entretoises radiales engagées dans les encoches en L et verrouiller ainsi la fraise. A sa seconde extrémité, le tube de manoeuvre comporte une bague de manoeuvre, par laquelle l'utilisateur peut déverrouiller la fraise en provoquant le coulissement du tube de manoeuvre et de la platine coulissante à l'écart de la douille à baïonnette.

D'autres fraises sont décrites dans les documents FR-A-2 281 095, EP-A-0 147 339, EP-A-0 327 509, US-A-4 712 951

Les outils décrits dans tous ces documents ont seulement une fonction de fraisage.

La seconde opération consiste à adapter une pièce d'essai dans la cavité osseuse creusée par la fraise, après enlèvement de la fraise. La pièce d'essai présente la forme de l'implant définitif qui devra ultérieurement être adapté dans la cavité. Ensuite, on retire la pièce d'essai et on adapte l'implant cotyloïdien, avec interposition de ciment, par exemple à l'aide d'un outil impacteur tel que décrit dans le document EP-A-0 470 912.

Selon une autre technique opératoire, décrite par exemple dans le document EP-A-0 051 359, après une première opération identique de réalisation de la cavité hémisphérique osseuse au moyen d'une fraise, on adapte une cupule métallique, enfoncée en force dans la cavité. On introduit dans la cupule métallique un noyau d'essai femelle, constituant une interface entre la cupule métallique et une tête de prothèse fémorale. On retire le noyau d'essai et on adapte enfin le noyau définitif par encliquetage.

Un premier inconvénient de ces techniques est la relative complexité de l'instrumentation, car il faut disposer de pièces d'essai spécifiques. Un autre inconvénient de ces techniques est que la stabilité de l'articulation définitive liée à l'orientation de l'implant reste aléatoire.

### EXPOSE DE L'INVENTION

Ainsi, un premier problème proposé par l'invention est de simplifier l'instrumentation, en permettant d'effectuer des manoeuvres d'essai de l'articulation avant fixation définitive de la prothèse, sans recourir à des pièces d'essai spécifiques distinctes.

Un autre problème proposé par l'invention est de réduire sensiblement les risques résiduels de luxation de l'articulation définitive. L'idée qui est à la base de l'invention est alors que ces risques résiduels de luxation résultent de la difficulté de contrôler et de fixer l'orientation relative de l'implant définitif par rapport au bassin et à la prothèse fémorale du patient. L'invention fournit ainsi des moyens permettant de choisir, de contrôler et de fixer aisément et efficacement l'orientation relative de l'implant définitif par rapport au bassin et à la prothèse fémorale du patient, en reproduisant fidèlement la position prise par les pièces lors des essais.

Pour atteindre ces objets ainsi que d'autres, une instrumentation de pose de prothèse de hanche selon l'invention comprend un porte-fraise et au moins une fraise adaptable de façon amovible sur le porte-fraise, la fraise comportant une calotte sensiblement hémisphérique creuse avec des lumières à bord coupant et une base ouverte dans laquelle sont prévus des moyens de fixation amovible du porte-fraise ;
- la calotte sensiblement hémisphérique comporte des lumières supplémentaires de dimension suffisamment grande pour permettre de contrôler le contact entre le fond de cavité cotyloïdienne creusée par la fraise et la surface externe hémisphérique de la fraise adaptée dans ladite cavité,
- la fraise comporte un noyau intérieur central solidaire de la calotte sensiblement hémisphérique, avec une face externe écartée de la paroi de calotte sensiblement hémisphérique pour laisser le passage des fragments d'os découpés par la fraise, et avec un creux hémisphérique concentrique ouvert selon la base de calotte et dont le diamètre intérieur est choisi pour correspondre au diamètre externe d'une tête de prothèse fémorale à poser,
- la fraise est associée à des moyens de fixation provisoire de la fraise dans la cavité cotyloidienne réalisée.

La fraise peut ainsi constituer en elle-même un cotyle d'essai, évitant d'avoir recours à une pièce d'essai spécifique.

Selon une réalisation avantageuse, le noyau intérieur central est solidarisé à la calotte sensiblement hémisphérique par au moins deux entretoises radiales disposées selon la base de la calotte, lesquelles entretoises radiales pouvant servir de moyens d'accrochage de la fraise sur le porte-fraise.

Selon un mode de réalisation, le noyau intérieur central est amovible, constitué d'une pièce rapportée adaptable dans la calotte. En alternative, le noyau intérieur central peut être solidaire de la calotte, non amovible.

De préférence, la calotte sensiblement hémisphérique comporte, à sa base, un rebord périphérique annulaire intérieur percé de trous divergents pour le passage de pointes de fixation provisoire.

Le porte-fraise peut avantageusement comprendre un manche dont une première extrémité comporte des moyens d'accrochage pour solidariser de façon amovible la fraise, et dont la seconde extrémité comporte un embout d'accouplement permettant un accouplement sélectif à l'arbre d'un moteur d'entraînement ou à une poignée de manipulation.

De préférence, l'instrumentation comprend une poignée de manipulation comportant des moyens de visée permettant d'orienter le porte-fraise et la fraise selon une orientation déterminée anatomique.

La poignée à moyens de visée peut avantageusement être adaptable également sur un manche d'impaction adapté pour recevoir et placer le cotyle définitif, de façon à orienter le cotyle définitif selon la même orientation que celle précédemment prise par la fraise. Une telle disposition permet de choisir, de contrôler et de fixer aisément et efficacement l'orientation relative du cotyle définitif par rapport au bassin et à la prothèse fémorale du patient, pour réduire les risques résiduels de luxation de l'articulation définitive.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :

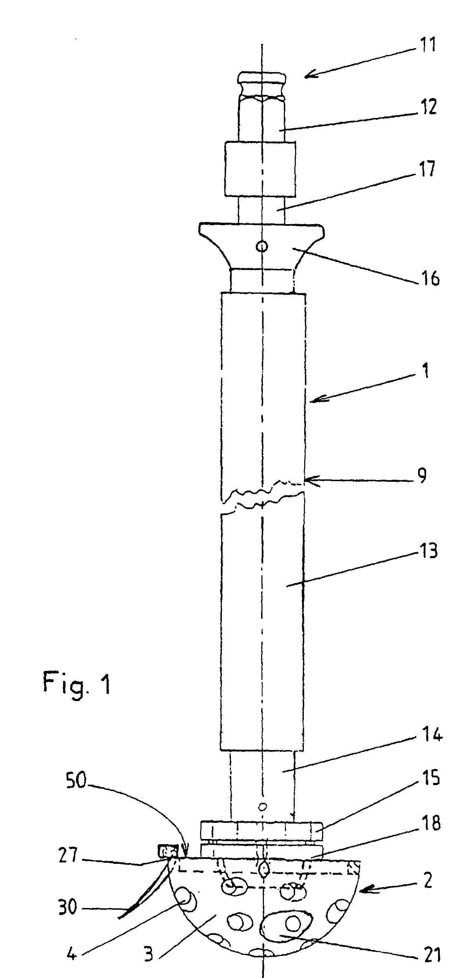

- la figure 1 est une vue de côté d'une fraise et d'un porte-fraise selon la présente invention, en position accouplée et verrouillée

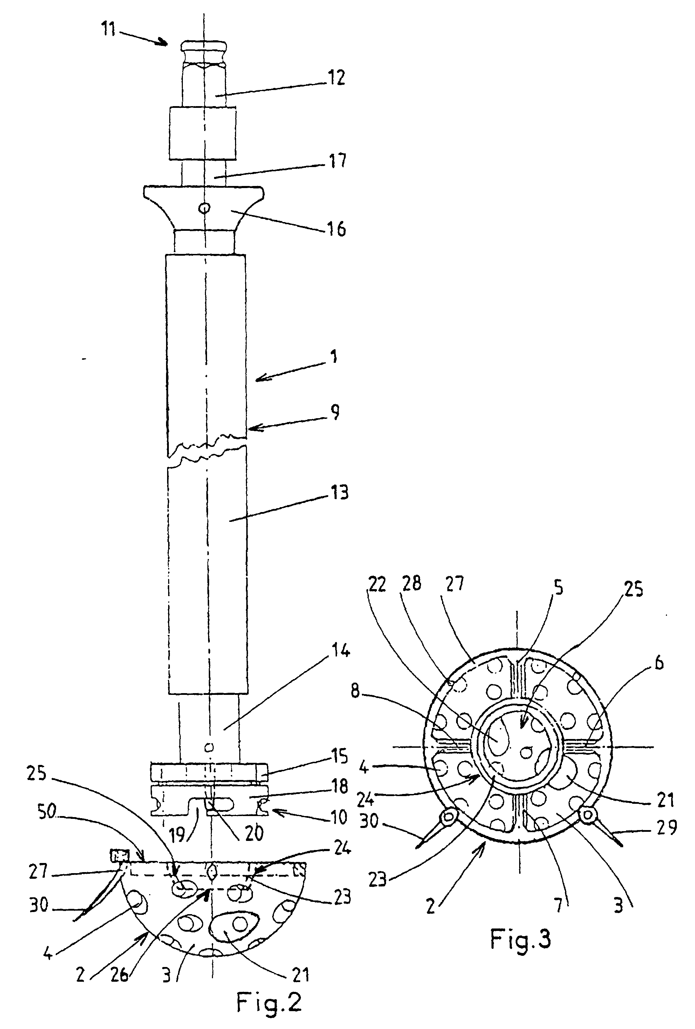

- la figure 2 est une vue de côté de l'ensemble fraise - porte-fraise de la figure 1, en position désaccouplée ;
- la figure 3 est une vue de dessus de la fraise des figures 1 et 2 ;
- la figure 4 est une vue de face de l'instrumentation de pose de prothèse de hanche selon un mode de réalisation de l'invention, en utilisation sur un patient placé en décubitus latéral ;
- la figure 5 est une vue de dessus de l'instrumentation et du patient dans la position de la figure 4 ; et
- la figure 6 est une vue de face du détail de l'articulation provisoire de hanche selon l'invention au cours de l'étape de vérification de la stabilité.

### DESCRIPTION DES MODES DE REALISATION PREFERES

Dans le mode de réalisation illustré sur les figures, une instrumentation de pose de prothèse de hanche selon l'invention comprend un porte-fraise 1 et au moins une fraise 2 adaptable de façon amovible sur le porte-fraise 1.

La fraise 2 comprend une calotte sensiblement hémisphérique 3 creuse, avec des lumières 4 à bord coupant et une base 50 ouverte dans laquelle sont prévus des moyens de fixation amovible du porte-fraise 1.

Dans la réalisation illustrée sur les figures, les moyens de fixation amovible du porte-fraise 1 comprennent des entretoises radiales 5, 6, 7 et 8 disposées selon la base de la calotte. Les entretoises radiales sont de préférence au moins au nombre de deux. Dans la réalisation illustrée, elles sont au nombre de quatre.

Le porte-fraise 1 comprend un manche 9 dont une première extrémité 10 comporte des moyens d'accrochage pour solidariser de façon amovible la fraise 2, et dont la seconde extrémité 11 comporte un embout d'accouplement 12 permettant un accouplement sélectif à l'arbre d'un moteur d'entraînement ou à une poignée de manipulation.

Le manche 9 du porte-fraise 1 comprend un tube protecteur 13 extérieur dans lequel tourillonne librement un tube de manoeuvre 14. Une première extrémité du tube de manoeuvre 14 porte une platine coulissante 15. La seconde extrémité du tube de manoeuvre 14 porte une bague de manoeuvre 16, accessible à l'utilisateur pour déplacer axialement le tube de manoeuvre 14.

Le manche 9 comprend en outre un arbre d'entraînement 17 central portant à sa première extrémité l'embout d'accouplement 12, et portant à sa seconde extrémité une douille de verrouillage 18. La douille de verrouillage 18 comporte, sur son bord extrême, des fentes en L telles que la fente 19, en nombre égal au nombre des entretoises radiales 5-B, et conformées pour constituer un système d'engagement à baïonnette pour l'adaptation de la fraise 2 sur 1a douille de verrouillage 18.

Le tube de manoeuvre 14 est monté à coulissement axial sur l'arbre d'entraînement 17, entre une position de verrouillage dans laquelle la platine coulissante 15 est à proximité de ou en appui contre la douille de verrouillage 18, et une position déverrouillée dans laquelle la platine coulissante 15 est à l'écart de la douille de verrouillage 18.

La platine coulissante 15 comporte des ergots longitudinaux tels que l'ergot 20, venant fermer chacune des fentes telles que la fente en L 19 lorsque la platine coulissante 15 est en position de verrouillage. Un ressort repousse le tube de manoeuvre 14 vers la douille de verrouillage 18. L'utilisateur peut déplacer le tube de manoeuvre 14 à l'encontre de la force exercée par ledit ressort, en tirant la bague de manoeuvre 16, pour dégager les ergots 20 hors des fentes en L 19 et autoriser le déverrouillage de la fraise 2.

Cette disposition est similaire de celle décrite dans le document EP-A-0 704 151, publié après la date de priorité.

La douille de verrouillage 18 présente un diamètre légèrement inférieur à celui de la fraise 2, pour occuper seulement la portion périphérique de la base 50 de fraise, laissant libre la partie centrale de la base 50.

Selon l'invention, la calotte 3 porte en outre des lumières supplémentaires telles que les lumières 21 et 22, de dimension suffisamment grande pour permettre à l'utilisateur de contrôler le contact entre le fond de cavité cotyloïdienne creusée par la fraise 2 et la surface externe hémisphérique de la fraise 2 adaptée dans ladite cavité.

En outre, la fraise 2 comporte un noyau intérieur central 23, solidaire de la calotte sensiblement hémisphérique 3, avec une face externe 24 écartée de la paroi de calotte sensiblement hémisphérique 3 pour laisser le passage des fragments d'os découpés par la fraise 2. Le noyau intérieur central 23 comporte en outre un creux hémisphérique 25 concentrique ouvert selon la base 50 de calotte et dont le diamètre intérieur est choisi pour correspondre au diamètre externe d'une tête de prothèse fémorale à poser. De préférence, le noyau intérieur central 23 est également ouvert à son sommet 26 opposé à la base 50 de calotte, pour laisser apparaître la portion centrale de calotte 3 et notamment les lumières 21 et 22, et pour favoriser l'évacuation des débris d'os lors du fraisage.

La fraise 2 est associée à des moyens de fixation provisoire de la fraise dans la cavité cotyloïdienne réalisée. Par exemple, comme illustré sur les figures, la calotte sensiblement hémisphérique 3 comporte, à sa base 50, un rebord 27 périphérique annulaire intérieur, percé de trous divergents 28 pour le passage de pointes de fixation provisoire, par exemple les pointes 29 et 30.

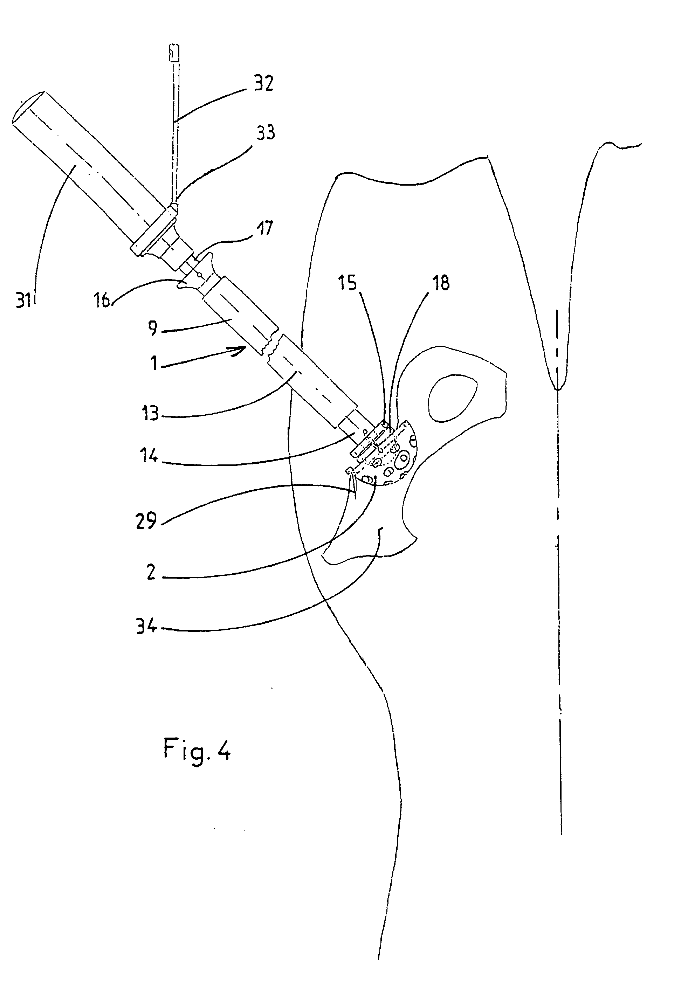

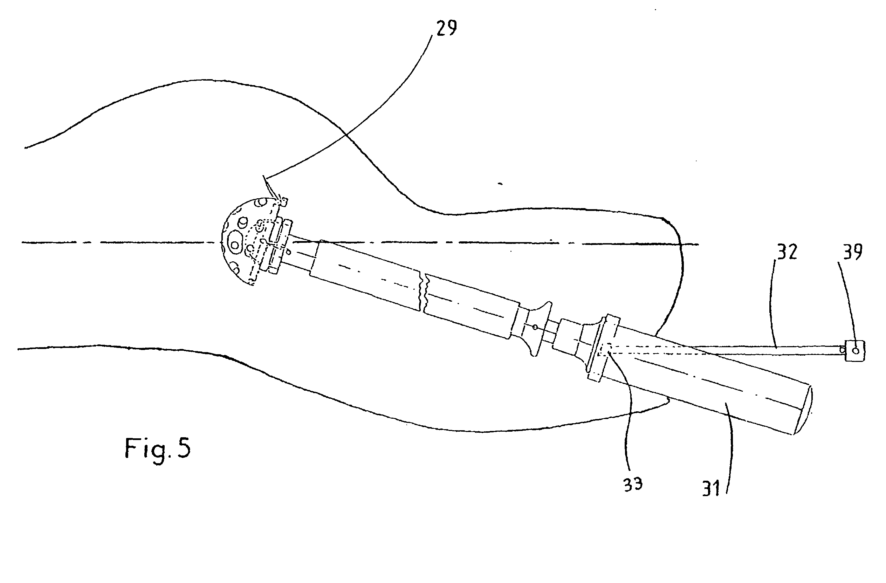

Comme illustré sur les figures 4 et 5, l'instrumentation selon l'invention comprend en outre une poignée de manipulation 31, adaptée pour s'accoupler sur l'embout 12 à l'extrémité 11 du porte-fraise 1. La poignée de manipulation 31 comprend avantageusement des moyens de visée permettant d'orienter le porte-fraise 1 et la fraise 2 selon une orientation déterminée anatomique.

Les moyens de visée comprennent une tige 32 articulée par sa première extrémité 33 sur la poignée de manipulation 31 et blocable sur la poignée 31 en toutes orientations dans une plage de réglage d'orientation 5 appropriée. En alternative, la tige 32 peut être orientée de façon fixe sur la poignée 31 selon une orientation faisant un angle approprié pour que la tige 32 soit horizontale lorsque l'axe longitudinal du porte-fraise 1 fait un angle de 45° avec le plan horizontal et un angle de 15° avec le plan vertical contenant la tige 32. Ces angles correspondent sensiblement à l'angle d'inclinaison verticale de 45° de l'implant cotyloïdien, et à l'angle de 15° d'antéversion de l'implant cotyloïdien.

De préférence, la tige 32 incorpore des niveaux à bulle 39 pour repérer l'orientation de la tige 32 dans le plan horizontal.

L'instrumentation comprend en outre un manche d'impaction, adapté pour recevoir et retenir le cotyle définitif à sa première extrémité et pour recevoir la poignée de manipulation 31 à sa seconde extrémité, ladite poignée 31 ayant les moyens de visée, de façon que l'utilisateur puisse orienter le cotyle définitif selon la même orientation que celle précédemment prise par la fraise 2.

L'instrumentation précédemment décrite peut être utilisée de la façon suivante, décrite en se référant aux figures 4 à 6.

Au cours d'une première étape, la fraise 2 est adaptée sur le porte-fraise 1, lequel est accouplé à un moteur non représenté entraînant l'arbre 17 en rotation axiale dans le tube de manoeuvre 14 et dans le tube protecteur 13. Le moteur entraîne ainsi en rotation la fraise 2 engagée et verrouillée sur la douille de verrouillage 18 du porte-fraise 1. On réalise ainsi une cavité hémisphérique dans l'os du bassin 34.

Ensuite, on désaccouple le moteur, et on engage en bout du porte-fraise 1 la poignée 31 de manipulation, avec sa tige de visée 32. Le patient étant en décubitus latéral, on oriente l'axe du porte-fraise 1 de telle façon que la tige 32 soit en position horizontale et alignée avec l'axe longitudinal du patient, comme illustré sur les figures 4 et 5. Ainsi, l'axe du porte-fraise 1 est incliné à 45° environ par rapport au plan horizontal comme illustré sur la figure 4, et à 15° environ par rapport à l'axe longitudinal du patient comme illustré sur la figure 5 en vue de dessus. En maintenant cette orientation du porte-fraise 1, on fixe alors 1a fraise 2 dans l'os du bassin 34 par enfoncement des clous tels que le clou 29.

Le porte-fraise 1 est alors dégagé de la fraise 2, par manoeuvre de la bague de manoeuvre 16, puis légère rotation du porte-fraise 1 et translation à l'écart de la fraise 2 pour dégager l'encliquetage à baïonnette.

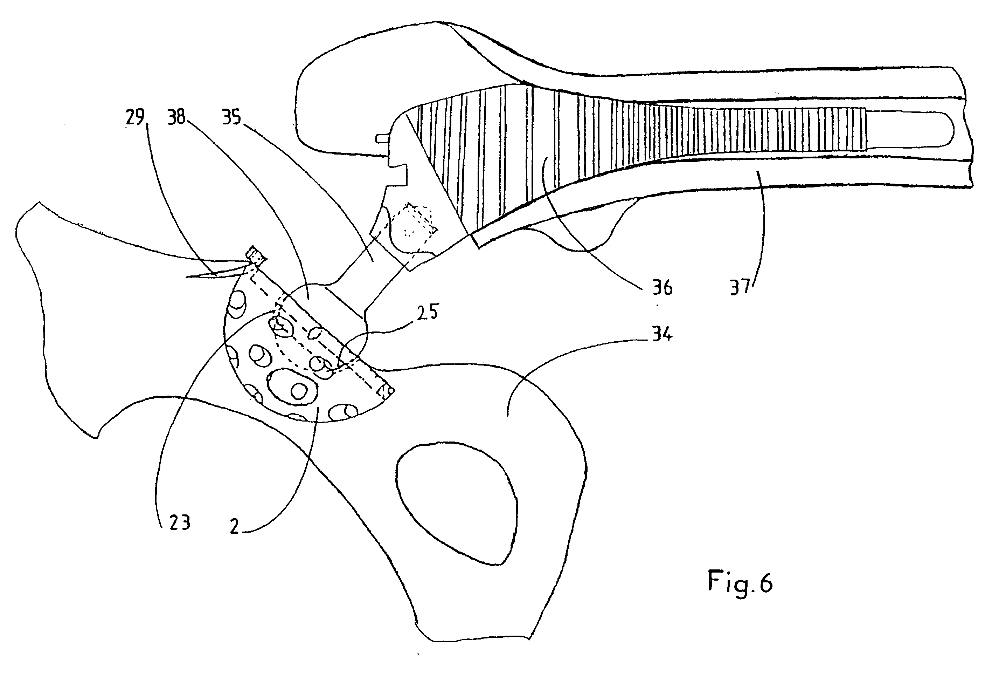

Ensuite, comme illustré sur la figure 6, la fraise 2 peut être utilisée comme cotyle d'essai, en plaçant une tête d'essai 35 au bout d'une râpe 36 adaptée pour creuser dans le fémur 37 la cavité destinée à recevoir la tige de prothèse fémorale. La fraise 2, par la présence du noyau 23 avec son creux hémisphérique 25, est appropriée pour recevoir elle-même l'extrémité sphérique 38 de la tête d'essai 35, constituant ainsi une articulation provisoire permettant de tester la bonne efficacité de l'articulation définitive, et notamment l'absence de risque de luxation. En effet, dans la disposition illustrée sur la figure 6, on peut donner au fémur 37 du patient toutes les orientations qui sont nécessaires lors des mouvements habituels du membre inférieur, et vérifier que la tête d'essai 35 reste engagée correctement dans la fraise 2. En cas de défaut, par exemple en cas de risque de luxation, on retire la tête d'essai 35, on place à nouveau sur la fraise 2 le porte-fraise 1 et la poignée de manipulation 31, on enlève les clous 29 et on modifie de façon appropriée l'orientation de la fraise 2 pour réduire les risques de luxation. On fixe à nouveau la fraise 2 à l'aide des clous 29. On repère alors la nouvelle orientation de la fraise 2, en repérant l'orientation du porte-fraise 1 par rapport à la tige 32. On enlève le porte-fraise 1 et l'on replace la tête d'essai 35 pour une nouvelle vérification des risques de luxation.

Lorsque les essais sont concluants, on enlève la tête d'essai 35 et la fraise 2, que l'on remplace par le cotyle définitif qui présente la même forme externe que la fraise 2, et qui comporte une cavité interne de même forme que le creux hémisphérique 25 de la fraise 2. Le cotyle définitif est placé à l'aide du manche d'impaction, adapté pour être solidaire du cotyle définitif à sa première extrémité et pour recevoir la poignée 31 à tige de visée 32 à sa seconde extrémité. Il est alors important d'orienter le manche d'impaction et le cotyle définitif dans la même orientation que celle précédemment prise par le porte-fraise 1 et la fraise 2, en plaçant la tige de visée 32 dans la même position que celle prise lors du dernier essai effectué avec la fraise 2. On s'assure ainsi que le cotyle définitif prend la même orientation que celle prise précédemment par la fraise 2 lors des essais satisfaisants de la tête d'essai 35. On assure alors la fixation définitive de l'implant définitif dans le cotyle osseux.

La présente invention n'est pas limitée aux modes de réalisation qui ont été explicitement décrits, mais elle en inclut les diverses variantes et généralisations contenues dans le domaine des revendications ci-après.

## Revendications

1. Instrumentation de pose de prothèse de hanche, comprenant un porte-fraise (1) et au moins une fraise (2) adaptable de façon amovible sur le porte-fraise (1), la fraise (2) comportant une calotte sensiblement hémisphérique (3) creuse avec des lumières (4) à bord coupant et une base (50) ouverte dans laquelle sont prévus des moyens de fixation amovible du porte-fraise (1), **caractérisée en ce que**
- la calotte sensiblement hémisphérique (3) comporte des lumières supplémentaires (21, 22) de dimension suffisamment grande pour permettre de contrôler le contact entre le fond de cavité cotyloidienne creusée par la fraise (2) et la surface externe hémisphérique de la fraise (2) adaptée dans ladite cavité,
- la fraise (2) comporte un noyau intérieur central (23) solidaire de la calotte sensiblement hémisphérique (3), avec une face externe (24) écartée de la paroi de calotte sensiblement hémisphérique (3) pour laisser le passage des fragments d'os découpés par la fraise (2), et avec un creux hémisphérique (25) concentrique ouvert selon la base (50) de calotte et dont le diamètre intérieur choisi pour correspondre au diamètre externe d'une tête de prothèse fémorale à poser,
- la fraise (2) est associée à des moyens de fixation provisoire (29, 30) de la fraise dans la cavité cotyloïdienne réalisée.

2. Instrumentation selon la revendication 1, **caractérisée en ce que** le noyau intérieur central (23) est solidarisé à la calotte sensiblement hémisphérique (3) par au moins deux entretoises radiales (5-8) disposées selon la base (50) de la calotte (3).

3. Instrumentation selon l'une des revendications 1 ou 2, **caractérisée en ce que** le noyau intérieur central (23) est amovible, constitué d'une pièce rapportée adaptable dans la calotte (3).

4. Instrumentation selon l'une quelconque des revendications 1 à 3, **caractérisée en ce que** la calotte sensiblement hémisphérique (3) comporte, à sa base (50), un rebord (27) périphérique annulaire intérieur percé de trous divergents (28) pour le passage de pointes de fixation provisoire (29, 30).

5. Instrumentation selon l'une quelconque des revendications 1 à 4, **caractérisée en ce que** le porte-fraise (1) comprend un manche (9) dont une première extrémité (10) comporte des moyens d'accrochage (18) pour solidariser de façon amovible la fraise (2), et dont la seconde extrémité (11) comporte un embout d'accouplement (12) permettant un accouplement sélectif à l'arbre d'un moteur d'entraînement ou à une poignée de manipulation (31).

6. Instrumentation selon la revendication 5, **caractérisée en ce qu'**elle comprend une poignée de manipulation (31) comportant des moyens de visée (32) permettant d'orienter le porte-fraise (1) et la fraise (2) selon une orientation déterminée anatomique.

7. Instrumentation selon la revendication 6, **caractérisée en ce que** les moyens de visée comprennent une tige (32) articulée par sa première extrémité (33) sur la poignée (31) de manipulation et blocable sur la poignée (31) de manipulation en toutes orientations dans une plage de réglage d'orientation appropriée, et incorporant des niveaux à bulle (39) pour repérer l'orientation de la tige (32) dans le plan horizontal.

8. Instrumentation selon l'une des revendications 6 ou 7, **caractérisée en ce qu'**elle comprend en outre un manche d'impaction, adapté pour recevoir et retenir le cotyle définitif à sa première extrémité et pour recevoir à sa seconde extrémité la poignée (31) à moyens de visée (32), de façon à orienter le cotyle définitif selon la même orientation que celle prise précédemment par la fraise (2).

## Patentansprüche

1. Instrument zum Einsetzen einer Hüftprothese mit einem Fräserträger (1) und mindestens einem Fräser (2), der unbeweglich an dem Fräserträger (1) anbringbar ist, wobei der Fräser (2) eine im wesentlichen halbkugelförmige hohle Kalotte (3) mit Öffnungen (4) mit Schneidrand und einer offenen Basis (50) aufweist, in welcher Mittel zur unbeweglichen Befestigung des Fräserträgers (1) vorgesehen sind, **dadurch gekennzeichnet, daß**
- die im wesentlichen halbkugelförmige Kalotte (3) zusätzliche Öffnungen (21, 22) aufweist, deren Dimension ausreichend groß ist, um den Kontakt zwischen dem Boden des Hohlraumes der Hüftgelenkspfanne, die durch den Fräser (2) ausgehöhlt ist, und der halbkugelförmigen Außenfläche des Fräsers (2), die in dem genannten Hohlraum anliegt, zu überwachen,
- der Fräser (2) einen inneren zentralen Kern (23) aufweist, der einstückig mit der im wesentlichen halbkugelförmigen Kalotte (3) ist, mit einer Außenfläche (24), die im Abstand zu der Wand der im wesentlichen halbkugelförmigen Kalotte (3) ist, um von dem Fräser (2) abgeschnittene Knochenfragmente passieren zu lassen, und mit einer halbkugelförmigen Ausnehmung (25), die längs der Basis (50) der Kalotte konzentrisch offen ist und deren Innendurchmesser so gewählt war, daß er dem Außendurchmesser eines Kopfes einer einzusetzenden Hüftprothese entspricht,
- der Fräser (2) mit Mitteln (29, 30) zur provisorischen Fixierung des Fräsers in dem geschaffenen Hohlraum für die Hüftgelenkpfanne versehen ist.

2. Instrument nach Anspruch 1, **dadurch gekennzeichnet, daß** der innere zentrale Kern (23) mittels mindestens zwei radialen Stegen (5-8), die an der Basis (50) der Kalotte (3) angeordnet sind, mit der im wesentlichen halbkugelförmigen Kalotte (3) einstückig verbunden ist.

3. Instrument nach einem der Ansprüche 1 oder 2, **dadurch gekennzeichnet, daß** der zentrale innere Kern (23) unbeweglich ist, was durch ein in die Kalotte (3) eingesetztes Teil erreicht wird.

4. Instrument nach einem der Ansprüche 1 bis 3, **dadurch gekennzeichnet, daß** die im wesentlichen halbkugelförmige Kalotte (3) an ihrer Basis (50) einen umlaufenden ringförmigen inneren Rand (27) aufweist, der von divergenten Löchern (28) durchbrochen ist für die Passage von provisorischen Befestigungspunkten (29, 30).

5. Instrument nach einem der Ansprüche 1 bis 4, **dadurch gekennzeichnet, daß** der Fräserträger (1) eine Haltestange (9) aufweist, deren erstes Ende (10) Haltemittel (18) trägt, um den Fräser (2) unbeweglich zu befestigen, und deren zweites Ende (11) ein Kupplungsstück (12) trägt, das wahlweise die Ankupplung der Welle eines Antriebsmotors oder eines Manipulationsgriffstückes (31) gestattet.

6. Instrument nach Anspruch 5, **dadurch gekennzeichnet, daß** es ein Manipulationsgriffstück (31) aufweist, das Beobachtungsmittel (32) trägt, die es gestatten, den Fräserträger (1) und den Fräser (2) gemäß einer vorbestimmten anatomischen Ausrichtung auszurichten.

7. Instrument nach Anspruch 6, **dadurch gekennzeichnet, daß** die Beobachtungsmittel einen Schaft (32) enthalten, der mit seinem ersten Ende (33) an dem Manipulationsgriffstück (31) angelenkt ist und in allen Ausrichtungen an dem Manipulationsgriffstück (31) in einem geeigneten Einstellbereich der Ausrichtung blockierbar ist, und eine Wasserwaage (39) enthält, um die Ausrichtung des Schaftes (32) in der horizontalen Ebene zu markieren.

8. Instrument nach einem der Ansprüche 6 oder 7, **dadurch gekennzeichnet, daß** es zusätzlich eine Stoßstange enthält, die so ausgebildet ist, daß sie die endgültige Hüftgelenkspfanne an ihrem ersten Ende aufnimmt und hält und an ihrem zweiten Ende das Griffstück (31) mit dem Beobachtungsmittel (32) aufnimmt, um die endgültige Hüftgelenkspfanne in der gleichen Orientierung auszurichten, die zuvor von dem Fräser (2) eingenommen wurde.

## Claims

1. Instruments for fitting hip prostheses, comprising a milling, tool support (1) and at least one milling tool (2) removably attachable to the milling tool support (1), the milling tool (2) having a hollow substantially hemispherical dome (3) with sharp-edged openings (4) and an open base (50) in which are provided means for removably fixing the milling tool support (1), **characterised in that** :
- the substantially hemispherical dome (3) includes additional openings (21, 22) of sufficiently large size to enable checking of contact between the bottom of the cotyloid cavity hollowed out by the milling tool. (2) and the hemispherical external surface of the milling tool (2) fitted into said cavity,
- the milling tool (2) includes a central interior core (23) attached to the substantially hemispherical dome (3), with an external face (24) separated from the wall of the substantially hemispherical dome (3) to leave a passage for bone fragments cut off by the milling tool (2), and with an open concentric hemispherical recess (25) at the base (50) of the dome the inside diameter of which has been chosen to correspond to the outside diameter of a femoral prosthesis head to be fitted,
- the milling tool (2) is associated with means (29, 30) for temporarily fixing the milling tool into the cotyloid cavity formed.

2. Instruments according to claim 1, **characterised in that** the central interior core (23) is attached to the substantially hemispherical dome (3) by at least two radial spacer members (5-8) disposed at the base (50) of the dome (3).

3. Instruments according to claim 1 or 2, **characterised in that** the central interior core (23) is removable, and constituted by an attached part that can be fitted in the dome (3).

4. Instruments according to any one of claims 1 to 3, **characterised in that** the substantially hemispherical dome (3) includes, at its base (50), an interior annular peripheral rim (27) with divergent holes (28) in it for temporary fixing spikes (29, 30).

5. Instruments according to any one of claims 1 to 4, **characterised in that** the milling tool support (1) comprises a handle (9) a first end (10) of which includes attachment means (18) for removably fastening the milling tool (2), and the other end (11) of which includes a coupling end-piece (12) enabling selective coupling to the shaft of a drive motor or to a manipulator handle (31).

6. Instruments according to claim 5, **characterised in that** they comprise a manipulator handle (31) including sighting means (32) for orienting the milling tool support (1) and the milling tool (2) with a particular anatomical orientation.

7. Instruments according to claim 6, **characterised in that** the sighting means comprise a rod (32) articulated at its first end (33) to the manipulator handle (31) and adapted to be locked to the manipulator handle (31) in all orientations within an appropriate orientation adjustment range, and incorporating spirit levels (39) showing the orientation of the rod (32) in the horizontal plane.

8. Instruments according to claim 6 or 7, **characterised in that** they further comprise an impaction handle, adapted to receive and to retain the final cotyl at its first end and to receive at its second end the handle (31) with sighting means (32), so as to orient the final cotyl with the same orientation as that previously assumed by the milling tool (2).
